(19) **Europäisches Patentamt / European Patent Office / Office européen des brevets**

(11) **EP 4 656 234 B1**

(12) # FASCICULE DE BREVET EUROPEEN

(45) Date de publication et mention
de la délivrance du brevet:
**01.07.2026 Bulletin 2026/27**

(21) Numéro de dépôt: **24315256.8**

(22) Date de dépôt: **31.05.2024**

(51) Classification Internationale des Brevets (IPC):
*A61N 1/362* (2006.01)    *A61N 1/365* (2006.01)
*A61N 1/37* (2006.01)    *A61N 1/372* (2006.01)
*A61N 1/375* (2006.01)

(52) Classification Coopérative des Brevets (CPC):
**A61N 1/3627; A61B 5/1118; A61B 5/29;
A61B 5/4836; A61B 5/6869; A61N 1/36542;
A61N 1/36578; A61N 1/3704; A61N 1/3706;
A61N 1/37205; A61N 1/3756;** A61B 2562/0219;
A61N 1/36507; A61N 1/36592

(54) **MODULE DE CALCUL D'UNE CONSIGNE DE RYTHME CARDIAQUE POUR UN STIMULATEUR CARDIAQUE IMPLANTABLE ASSERVI À L'ACTIVITÉ D'UN PATIENT**

MODUL ZUR BERECHNUNG EINES HERZRHYTHMUSSOLLWERTS FÜR EINEN IMPLANTIERBAREN HERZSCHRITTMACHER, DER AN DIE AKTIVITÄT EINES PATIENTEN GESTEUERT WIRD

MODULE FOR CALCULATING A HEART RATE SETPOINT FOR AN IMPLANTABLE HEART STIMULATOR FOLLOWING THE ACTIVITY OF A PATIENT

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(43) Date de publication de la demande:
**03.12.2025 Bulletin 2025/49**

(73) Titulaire: **CAIRDAC**
**92160 Antony (FR)**

(72) Inventeur: **Makdissi, Alaa**
**75011 Paris (FR)**

(74) Mandataire: **Dupuis-Latour, Dominique**
**Bardehle Pagenberg SPE SAS**
**SO Square Opéra**
**5, rue Boudreau**
**75009 Paris (FR)**

(56) Documents cités:
EP-B1- 3 265 172    US-A1- 2020 147 396
US-B2- 11 045 657    US-B2- 9 724 518

**Description**

CONTEXTE DE L'INVENTION

*Domaine de l'invention*

L'invention concerne les dispositifs médicaux implantés, en particulier les stimulateurs cardiaques qui surveillent en continu le rythme du patient et délivrent en tant que de besoin au cœur des impulsions électriques de stimulation pour pallier un trouble du rythme sinusal du myocarde. L'invention est applicable de façon particulièrement avantageuse - mais non limitative - aux dispositifs implantables autonomes de type "capsule leadless", qui sont des implants dépourvus de toute liaison physique (*lead*) à un dispositif distant.

La capsule comprend divers circuits électroniques, capteurs, etc., ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule ou la paroi interne de l'oreillette.

L'un des aspects critiques de ces dispositifs miniaturisés est celui de l'autonomie électrique, et par voie de conséquence celui de la consommation des circuits électroniques, qui doit être la plus réduite possible.

Avec un implant leadless, compte tenu de ses très faibles dimensions, il n'est pas possible d'utiliser une batterie conventionnelle, même à forte densité. Aussi, il est prévu un système d'auto-alimentation, avec un récupérateur d'énergie de type "harvester" qui recueille l'énergie mécanique résultant des mouvements divers subis par le corps de l'implant au rythme des battements cardiaques, et convertit cette énergie mécanique en énergie électrique au moyen d'un transducteur approprié, pour assurer la recharge d'une batterie intégrée et l'alimentation des divers circuits et capteurs du dispositif. Ce système d'alimentation permet au dispositif de fonctionner en parfaite autonomie électrique pendant toute sa durée de vie, d'environ 8 à 10 ans.

Le WO 2019/001829 A1 (Cairdac) décrit un exemple d'une telle capsule leadless intracardiaque autonome munie d'un récupérateur d'énergie intégré.

L'invention n'est toutefois pas limitée à ce type particulier d'implant ; elle est applicable aussi bien à d'autres types de stimulateurs cardiaques alimentés par une batterie dont il faut préserver la durée de vie en minimisant la consommation électrique d'ensemble du dispositif.

En ce qui concerne la stimulation, celle-ci est généralement opérée en mode VVIR ou "rate-responsive", c'est-à-dire qu'en cas de rythme stimulé et non plus spontané (sinusal), la fréquence des impulsions de stimulation doit être modulée en fonction de l'activité physique du patient, avec une fréquence moindre lorsque le patient est inactif et une fréquence plus élevée pour une activité physique accrue. La fréquence du rythme stimulé ainsi asservi variera entre une fréquence minimale dite "fréquence de base" ($HR_{min}$) et la fréquence maximale ($HR_{max}$) propre au patient, qui définit un plafond pour la fréquence de stimulation calculée par les algorithmes d'asservissement.

L'asservissement de la fréquence de stimulation doit par ailleurs être le plus physiologique possible, en répliquant le rythme sinusal d'un cœur sain avec (i) une montée rapide du rythme en cas d'augmentation soudaine de l'activité (le patient qui se lève et marche, qui monte un escalier, etc.), et (ii) ensuite un retour au contraire très progressif et lent à la fréquence de base, de l'ordre de plusieurs dizaines de secondes ou plusieurs minutes après la détection d'une activité moindre (le patient qui a fini de monter l'escalier mais qui doit encore récupérer de son effort, etc.). Le taux de variation de la fréquence de stimulation asservie, c'est-à-dire la forme (linéaire ou non) de la caractéristique fréquence/effort, peut également être adapté à l'activité physique du patient.

Pour réaliser cette fonction d'asservissement, le niveau d'activité physique instantanée du patient est typiquement mesuré par un capteur dit "capteur d'activité ou "capteur G" qui est typiquement un accéléromètre, le plus souvent un accéléromètre 3D.

Ce type de capteur, qui délivre un signal accélérométrique avec des variations très rapides et de très grande amplitude, est à distinguer des capteurs de type "capteur physiologique" ou "capteur d'effort" tels que les capteurs de ventilation-minute ou "capteurs MV", qui délivrent un signal à variation lente représentatif des besoins métaboliques du patient, et qui ne sont d'ailleurs pas applicables au cas une capsule leadless autonome (ils reposent en effet sur la mesure d'une impédance transthoracique entre l'extrémité d'une sonde intracardiaque et un boitier distant d'un générateur de stimulateur).

*Description de la technique antérieure*

Le problème de l'invention est celui du calcul en continu (cycle-à-cycle) de la fréquence optimale des impulsions de stimulation que doit délivrer le dispositif, cette fréquence devant être calculée idéalement à chaque cycle cardiaque afin d'en optimiser l'adaptation à l'activité du patient.

Le US 2020/0147396 A1 (Shelton et al./Medtronic) décrit un stimulateur cardiaque leadless muni d'un capteur accélérométrique détectant les mouvements du patient et son niveau d'activité. Le signal du capteur accélérométrique est échantillonné et filtré de manière appropriée, puis traité de manière à déterminer à chaque nouveau cycle cardiaque une fréquence cible pour l'asservissement du dispositif.

Pour réduire la consommation du microprocesseur intégré qui effectue ces opérations, celui-ci n'est rendu actif que pendant une période d'échantillonnage de durée restreinte, après expiration d'une période de blanking pendant laquelle l'échantillonnage est suspendu.

Cette solution n'est toutefois pas optimale. En effet :

- à chaque cycle cardiaque, le réveil du processeur du mode dormant au mode actif n'est pas immédiat : il nécessite plusieurs dizaines de cycles de processeur, soit quelques microsecondes à quelques millisecondes avant de pouvoir commencer à recalculer la fréquence de stimulation ;
- pendant cette phase de réveil, la consommation du processeur est au moins équivalente à sa consommation pendant la phase active, alors même qu'il n'est pas encore capable d'échantillonner et de traiter le signal ;
- l'algorithme se base sur un cumul de 1000 valeurs antérieures de la fréquence de stimulation (soit une durée d'environ 15 minutes) pour tenir compte de l'hystérésis, c'est-à-dire du fait que la loi de variation n'est pas la même dans une situation d'augmentation de l'activité du patient (nécessitant une accélération rapide de la fréquence de simulation) et dans le cas d'une décroissance de cette activité (nécessitant un retour très progressif et lent à la fréquence de base). Cette analyse rétrospective sur une longue durée nécessite d'exécuter environ 2000 cycles de processeur avant de pouvoir stabiliser l'asservissement, entrainant un quasi-doublement de la consommation d'un algorithme asservi (VVIR) par rapport à un algorithme simple, non asservi (VVI) ;
- enfin, la présence d'une période de blanking fait qu'une partie du signal accélérométrique reste masquée et ne sera jamais prise en compte pour le calcul de l'asservissement, réduisant d'autant la précision de ce dernier.

Le but de l'invention est de pallier ces inconvénients et limitations en proposant une technique d'adaptation en continu du rythme cardiaque, avantageusement applicable à un implant leadless, n'imposant qu'une très faible consommation des circuits internes du dispositif, sans pour autant dégrader les performances de l'asservissement par rapport aux techniques connues.

RÉSUMÉ DE L'INVENTION

Pour résoudre les différents problèmes et atteindre les buts exposés ci-dessus, l'invention propose, essentiellement, un module de calcul d'une valeur de rythme cardiaque comprenant, de manière en elle-même connue, un module de calcul d'une valeur de consigne de rythme cardiaque, HR, ou d'intervalle cardiaque, RR, destinée à asservir à l'activité d'un patient la fréquence des impulsions de stimulation délivrées par un dispositif médical implantable actif, dans lequel la valeur HR ou RR de consigne est déterminée en continu par traitement numérique d'un signal d'activité échantillonné représentatif de l'activité instantanée du patient.

De façon caractéristique de l'invention, ce module comprend en outre :

- un étage de conversion, recevant en entrée une valeur courante du signal d'activité échantillonné et délivrant en sortie une première valeur de HR, ou de RR, cible obtenue par application d'une fonction activité/rythme cardiaque, ou respectivement activité/intervalle cardiaque, prédéterminée à une valeur courante du signal d'activité échantillonné ;
- un premier étage de filtrage numérique passe-bas, comprenant un filtre récursif apte à calculer, sur une première durée prédéterminée, une première moyenne mobile de la première valeur de HR ou RR cible délivrée par l'étage de conversion, donnant en sortie une seconde valeur de HR ou RR cible ; et
- un étage combineur, recevant en entrée (i) la première valeur de HR ou RR cible délivrée par l'étage de conversion, et (ii) la seconde valeur de HR ou RR cible délivrée par le premier étage de filtrage numérique passe-bas, et apte à déterminer le maximum des deux valeurs de HR cible, ou respectivement le minimum des deux valeurs de RR cible, reçues en entrée, donnant en sortie la valeur HR ou RR de consigne, pour asservir la fréquence de stimulation à l'activité du patient.

Selon diverses caractéristiques subsidiaires avantageuses :

- le module est intégré à un circuit ASIC ;
- le module comprend un microcontrôleur opérant sans mise en sommeil entre deux cycles cardiaques consécutifs ;
- le module comprend en outre un second étage de filtrage numérique passe-bas, comprenant un filtre récursif apte à calculer, sur une seconde durée prédéterminée inférieure à la première durée prédéterminée, une seconde moyenne mobile de la première valeur de HR ou RR cible, délivrée par l'étage de conversion, de manière à éliminer des composantes de bruit haute fréquence parasites non représentatives de l'activité instantanée du patient, l'étage

3

combineur et le premier étage de filtrage numérique passe-bas recevant en entrée la première valeur de HR ou RR cible, après que celle-ci a été filtrée par le second étage de filtrage numérique passe-bas ;
- la fonction activité/rythme cardiaque, ou respectivement la fonction activité/intervalle cardiaque, prédéterminée, est une fonction linéaire ;
- pour déterminer le temps de récupération du patient après une période d'activité intense, la première durée prédéterminée pour le calcul de la première moyenne mobile par le premier étage de filtrage numérique passe-bas est comprise entre 120 et 600 secondes ;
- dans ce dernier cas, pour déterminer la réactivité de l'adaptation du rythme cardiaque ou de l'intervalle cardiaque tout en éliminant les bruits d'activité de très courte durée, la seconde durée prédéterminée pour le calcul de la première moyenne mobile par le second étage de filtrage numérique passe-bas est comprise entre 1 et 2 secondes ;
- le filtre récursif du premier et/ou du second étage de filtrage numérique passe-bas est un filtre récursif exponentiel du 1er ordre ; et/ou
- le filtre récursif du premier et/ou du second étage de filtrage passe-bas est un filtre apte à calculer une moyenne mobile sans division ni multiplication numérique, en particulier un filtre apte à opérer par décalage de bits de la représentation numérique de la valeur de HR ou RR cible en entrée de l'étage de filtrage.

L'invention porte également sur un dispositif médical actif de type capsule autonome implantable logeant, dans un corps de dispositif, un ensemble électronique comprenant :

- un circuit de stimulation, apte à délivrer des impulsions de stimulation ;
- un circuit apte à délivrer un signal représentatif de l'activité instantanée d'un patient porteur du dispositif ;
- un module d'échantillonnage et d'extraction d'un signal d'activité échantillonné à partir du signal représentatif de l'activité instantanée du patient ; et
- un module de calcul d'une valeur HR ou RR de consigne tel que ci-dessus, pour asservir à l'activité du patient la fréquence des impulsions délivrées par le circuit de stimulation.

DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.

La Figure 1 illustre un dispositif médical de type capsule leadless dans son environnement, implanté au fond du ventricule droit d'un patient.
La Figure 2 présente sous forme schématique les principaux blocs fonctionnels constitutifs de la capsule leadless.
Les Figures 3a et 3b illustrent la manière d'asservir le rythme cardiaque d'activité du patient de la manière la plus physiologique possible, de façon comparable à l'adaptation naturelle chez un patient sain.
La Figure 4 est une représentation schématique, sous forme de blocs, des différents étages de traitement numérique du signal d'un module selon l'invention permettant de délivrer, à partir d'un signal d'activité échantillonné issu d'un accéléromètre, une valeur de consigne de rythme cardiaque propre à être appliquée en entrée d'un étage d'asservissement du circuit de stimulation d'un dispositif implanté.
Les Figures 5a à 5d sont des chronogrammes illustrant les valeurs des signaux échantillonnés relevées à différents niveaux du module selon l'invention illustré Figure 4, lors de variations détectées de l'activité du patient.
La Figure 6 est une caractéristique rythme cardiaque vs. niveau d'activité, correspondant aux variations d'activité illustrées aux Figures 5a à 5d.

DESCRIPTION DÉTAILLÉE DE MODES DE RÉALISATION

PRÉFÉRENTIELS DE L'INVENTION

On va maintenant décrire un exemple de réalisation du dispositif de l'invention, dans une application à une capsule implantable autonome destinée à être implantée dans une cavité cardiaque.
Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant ou non un récupérateur d'énergie de type PEH.

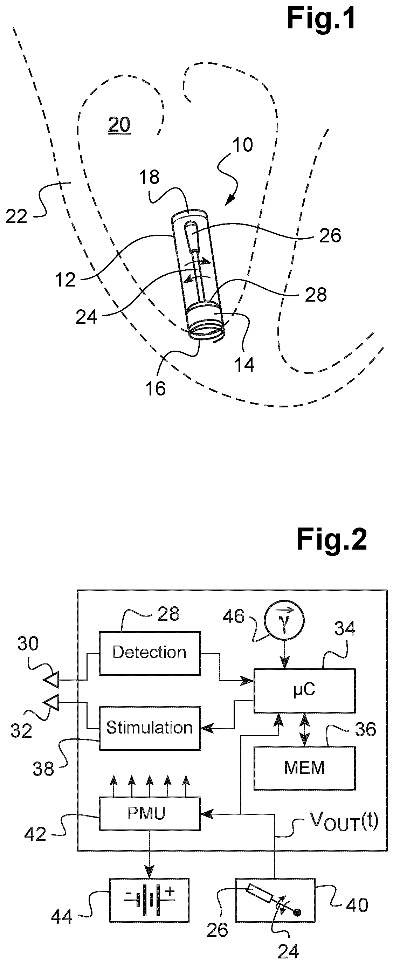

Sur la Figure 1, on a représenté un dispositif de type capsule leadless 10 dans une application à la stimulation cardiaque.
La capsule 10 se présente sous forme extérieure d'un implant avec un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en œuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en œuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises parles tissus cardiaques.

L'ensemble pendulaire est constitué d'une lame piézoélectrique 24 encastrée à l'une de ses extrémités et dont l'extrémité opposée, libre, est couplée à une masse inertielle mobile 26. La lame piézoélectrique 24 est une lame flexible élastiquement déformable qui constitue avec la masse inertielle 26 un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation.

La Figure 2 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer en tant que de besoin au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 24 et la masse inertielle 26 décrits plus haut en référence à la Figure 1.

La lame piézoélectrique 24, qui assure une fonction de transducteur mécano-électrique, convertit en charges électriques les sollicitations mécaniques subies et produit un signal électrique variable $V_{OUT}(t)$, qui est un signal alternatif oscillant à la fréquence d'oscillation libre de l'ensemble pendulaire lame 24/masse 30, et au rythme des battements successifs du myocarde auquel la capsule est couplée. Ce signal électrique variable $V_{OUT}(t)$ est délivré à un circuit de gestion de l'énergie ou PMU 42, qui redresse et régule le signal $V_{OUT}(t)$ de manière à produire en sortie une tension ou un courant continus stabilisés servant à l'alimentation des divers circuits électroniques et à la recharge d'une batterie intégrée 44.

La capsule leadless intègre également un capteur d'activité cardiaque 46 tel qu'un accéléromètre 1D, ou de préférence 3D, de type piézoélectrique, piézorésistif ou capacitif de type MEMS.

Le capteur 46 délivre en continu un signal composite contenant (i) des composantes représentatives de l'activité instantanée du patient porteur du dispositif et (ii) des composantes représentatives de l'accélération, due aux battements cardiaques, de la paroi sur laquelle est implanté la capsule.

Après échantillonnage et traitement, ce signal accélérométrique sera utilisé pour asservir à l'activité du patient la fréquence des impulsions de stimulation délivrées par le circuit de stimulation 38 (stimulation asservie de type VVIR), et/ou pour effectuer un test de capture, c'est-à-dire détecter la présence ou l'absence d'une contraction du myocarde consécutive à l'application d'une impulsion de stimulation.

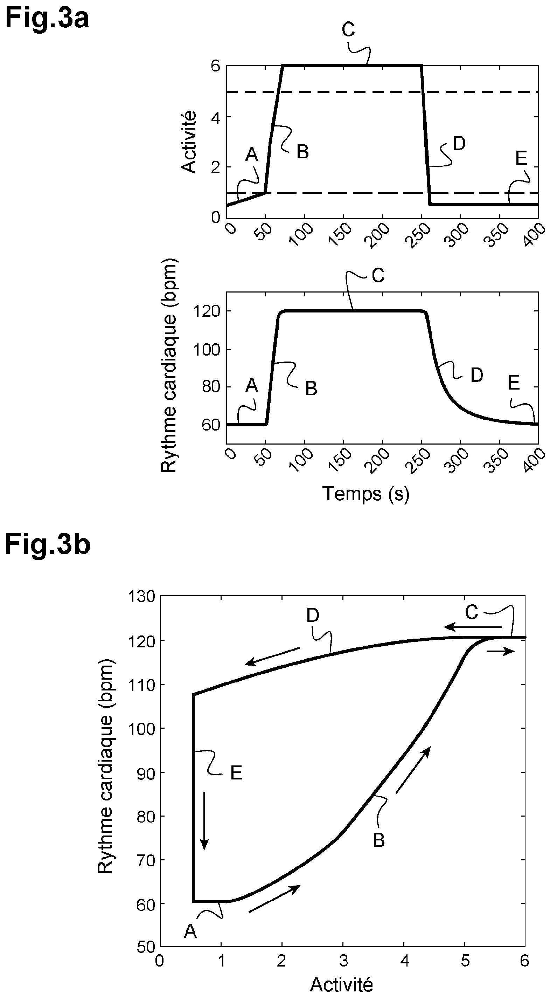

Sur les Figures 3a et 3b on a illustré la manière d'asservir le rythme cardiaque d'activité du patient de la manière la plus physiologique possible, de façon comparable à l'adaptation naturelle chez un patient sain.

La Figure 3a est un chronogramme montrant, en haut, un exemple de variation dans le temps de l'activité d'un patient, cette activité étant représentée par un indicateur en unités arbitraires variant de 0 à 5. En bas, la Figure 3a montre la manière dont le cœur s'adapte chez un patient sain à des variations subites d'activité, notamment le fait que la réponse à un début d'activité est différente de la réponse à un arrêt d'activité. Par exemple, en partant d'une situation au repos A à 60 bpm (fréquence de base $HR_{min}$ au repos) , une augmentation soudaine d'activité B provoque une augmentation très rapide du rythme cardiaque, dans cet exemple de 60 à 120 bpm (120 bpm étant dans cet exemple la fréquence cardiaque maximale $HR_{max}$ du patient), avec un temps de réponse de l'ordre de 2 à 10 secondes. Pendant la poursuite d'une activité soutenue, en C, le rythme cardiaque se maintient à un niveau élevé, dans cet exemple au rythme maximal $HR_{max}$. Lorsque l'activité décroît soudainement, en D, la baisse du rythme cardiaque est régulière et progressive jusqu'au retour à la fréquence de base $HR_{min}$, en E, avec un temps de réponse de l'ordre de 3 à 10 minutes.

Cette hystérésis de la réponse est visible notamment sur le diagramme paramétrique activité vs. rythme cardiaque de la Figure 3b, correspondant aux valeurs données en exemple à la Figure 3a.

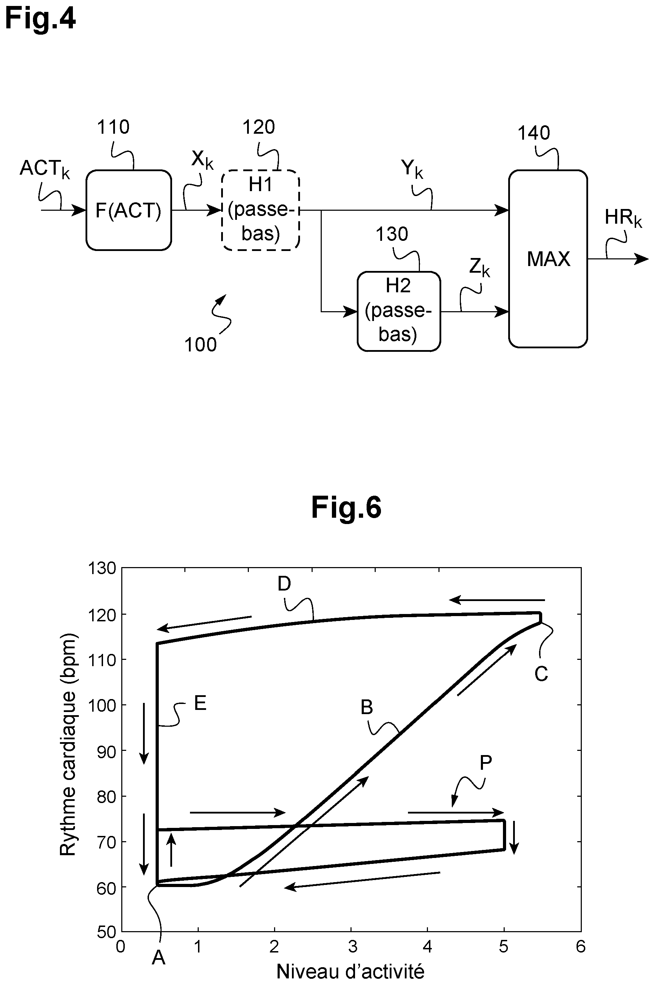

La Figure 4 est une représentation schématique, sous forme de blocs, des différents étages de traitement numérique du signal d'un module selon l'invention, destiné à délivrer une valeur de consigne de rythme cardiaque propre à être appliquée en entrée d'un étage d'asservissement d'un circuit de stimulation d'un dispositif implanté.

Le module 100 selon l'invention reçoit en entrée un signal d'activité échantillonné $ACT_k$. Ce signal d'activité échantillonné est issu d'un module de détection et de numérisation qui ne fait pas partie de la présente invention, et qui peut être d'un type en lui-même connu, ne nécessitant aucune adaptation particulière pour la mise en œuvre de la présente invention.

Le niveau d'activité d'un patient étant un paramètre qui varie dans une plage de fréquences relativement basses, de l'ordre de 1 à 7 Hz, la cadence d'échantillonnage peut être relativement faible, de l'ordre de 4 Hz, soit 4 échantillons par seconde, ou moins.

La numérisation du signal d'activité donne une métrique qui est quantifiée par un nombre limité de valeurs entières, par exemple une métrique sur 4 niveaux ou 12 niveaux élémentaires.

A l'instant t = k, l'échantillon $ACT_k$ du signal d'activité est appliqué en entrée d'un étage de conversion 110 qui applique à la valeur courante $ACT_k$ une fonction prédéterminée activité/rythme cardiaque F(ACT) et délivre en sortie une première valeur de rythme cardiaque cible $X_k$.

Le rythme cardiaque cible devant être nécessairement compris entre la valeur minimale $HR_{min}$ (fréquence de base) et une valeur maximale $HR_{max}$, dans le cas d'une fonction activité/rythme cardiaque linéaire la fonction F est de la forme :

$$X_k = \text{MAX} \{ HR_{min}, \text{MIN} (a.ACT_k + b.HR_{max}) \}.$$

Les valeurs de la pente a et de l'ordonnée à l'origine b sont spécifiques à chaque patient, et peuvent être des paramètres configurables par le médecin au moment de l'implantation ou lors d'une visite de contrôle.

En variante, la fonction F peut être une fonction non linéaire, monotone (comme dans l'exemple de l'arc de courbe AC de la Figure 3b correspondant à la réponse naturelle d'un cœur chez un patient sain), ou encore définie par des segments successifs, éventuellement paramétrables par le médecin, par exemple au moyen d'une interface graphique sur une tablette au moment de l'implantation ou d'une visite de contrôle.

La fonction rythme cardiaque/activité F de l'étage de conversion 110 définit une première valeur de rythme cardiaque cible $X_k$, mais ne définit pas l'intervalle de temps nécessaire pour atteindre cette première valeur cible $X_k$. Comme indiqué plus haut, pour répliquer la réponse du cœur d'un patient sain, il est souhaitable :

(i) que le rythme cardiaque augmente immédiatement après le début de l'activité. Un exemple typique est celui d'un patient assis, qui ensuite se lève et commence à monter un escalier : il est indispensable que le rythme cardiaque augmente très rapidement dans un tel cas de figure. Le temps de réponse pour atteindre la valeur cible de rythme cardiaque sera dans ce cas typiquement de l'ordre de 2 s à 10 s, selon le mode de vie du patient (pour un patient avec un mode de vie actif, le temps de réponse devra être beaucoup plus faible que dans le cas contraire) ;

(ii) que le rythme cardiaque diminue lentement et régulièrement après l'arrêt de l'activité, avec une constante de temps typique de l'ordre de 2 mn à 10 mn ; et

(iii) d'assurer une immunité à l'encontre des bruits non significatifs détectés par l'accéléromètre du circuit de détection d'activité. En particulier, des signaux dans une plage de fréquences élevées, ou des modifications brusques mais transitoires du niveau d'activité ne doivent pas conduire à un changement rapide du rythme cardiaque. Par exemple, dans le cas d'un patient qui se retourne dans son lit, ou qui tousse, son rythme cardiaque ne doit pas être modifié, ou pas significativement modifié, alors même que ces épisodes produiront une brusque, mais brève, variation de l'activité détectée par l'accéléromètre.

Pour répondre à ces diverses exigences, la valeur de rythme cardiaque cible $X_k$ va faire l'objet d'un traitement spécifique de filtrage, détaillé ci-dessous, opéré par les étages 120 à 140.

L'étage 120 est un étage de filtrage numérique passe-bas H1 destiné à éliminer les bruits de haute fréquence évoqués plus haut. Avantageusement, le filtre H1 est un filtre exponentiel récursif du premier ordre, calculant la moyenne des valeurs $X_k$ sur une durée $\tau_1$ de l'ordre de 1 ou 2 s, selon la cadence d'échantillonnage de la détection d'activité. Dans une mise en œuvre simple, et donc économe du point de vue de la consommation électrique des circuits, l'équation récursive du filtrage H1 peut être effectuée sans division ni multiplication, par exemple par division par 8 du signal : en effet, une telle division correspond à un simple décalage d'un bit à gauche de la valeur numérique du signal $X_k$.

Plus précisément, la sortie du filtre récursif H1 est de la forme :

$$Y_k = (1\text{-}\alpha_1).Y_{k\text{-}1} + \alpha_1.X_k,$$

$Y_k$ étant la valeur actuelle de $Y_k$ et $Y_{k\text{-}1}$ étant la valeur précédente dans la séquence d'échantillons.

Dans une division par 8, avec une cadence d'échantillonnage de 4 Hz (4 échantillons par seconde) et une moyenne calculée sur $\tau_1$ = 2 s, la pondération $(1 - \alpha_1)$ sera de 7/8 et celle de $\alpha_1$ de 1/8.

Lorsque l'échantillon d'activité suivant est acquis, la mise à jour de la valeur Y par la nouvelle valeur X peut être réalisée en deux opérations :

$$Y = M*Y - Y+X,$$

et

$$Y = Y/M,$$

où M est une valeur entière, arrondie, de $F_s \times \tau_1$, $F_s$ étant la fréquence d'échantillonnage de la détection d'activité et $\tau_1$ étant la constante prédéterminée, par exemple $\tau_1$ = 1 ou 2 s.

Si l'on prend pour M une puissance de 2 (M = 2^N), le calcul peut être réalisé simplement par la manipulation de bits suivante :

$$Y = (Y << N) - Y + X,$$

et

$$Y = (Y >> N),$$

où Y << N représente la valeur binaire de Y décalée vers la gauche de N bits (ce qui équivaut à multiplier Y par M), et Y >> N représente la valeur binaire de Y décalée vers la droite de N bits (ce qui équivaut à diviser Y par M).

La valeur cible $Y_k$ délivrée en sortie de l'étage 12 est appliquée à un étage 130, qui est un étage de filtrage passe-bas H2 destiné à prendre en compte l'hystérésis des épisodes de décroissance de l'activité. Avantageusement le filtre H2 est un filtre exponentiel récursif du premier ordre, calculant une moyenne mobile du signal $Y_k$ appliqué en entrée, avec une constante de temps $\tau_2$ longue, par exemple de 120 s, 300 s ou 600 s (valeur correspondant à la durée du temps de décroissance lorsque cesse l'activité).

Le signal de sortie $Z_k$ du filtre H2 de l'étage 130 est donné par :

$$Z_k = (1 - \alpha_2).Z_{k\text{-}1} + \dot{}\alpha_2.Y_k.$$

Z représente la moyenne mobile de Y sur une durée $\tau_2$, et peut être calculée par simple décalages de bits de la même manière que ce qui a été exposé plus haut pour le filtrage H1, sans multiplications ni divisions coûteuses en termes de consommation du processeur.

Une fois ces deux filtrages H1 et H2 opérés, on dispose en sortie des étages 120 et 130 de deux valeurs filtrées, respectivement $Y_k$ et $Z_k$, qui sont appliquées simultanément en entrée d'un étage 140.

L'étage 140 est un étage combineur qui délivre en sortie la plus élevée des deux valeurs $Y_k$ et $Z_k$ :

$$HR_k = MAX(Y_k, Z_k).$$

$HR_k$ sera la valeur cible finale destinée à servir de consigne pour l'asservissement du rythme cardiaque par le circuit de délivrance des impulsions de stimulation.

La valeur $HR_k$ ainsi obtenue est une valeur de consigne lissée et différenciée selon que l'on se situe dans une phase d'augmentation de l'activité (dans ce cas $HR_k = Y_k$) ou de baisse d'activité (dans ce cas $HR_k = Z_k$).

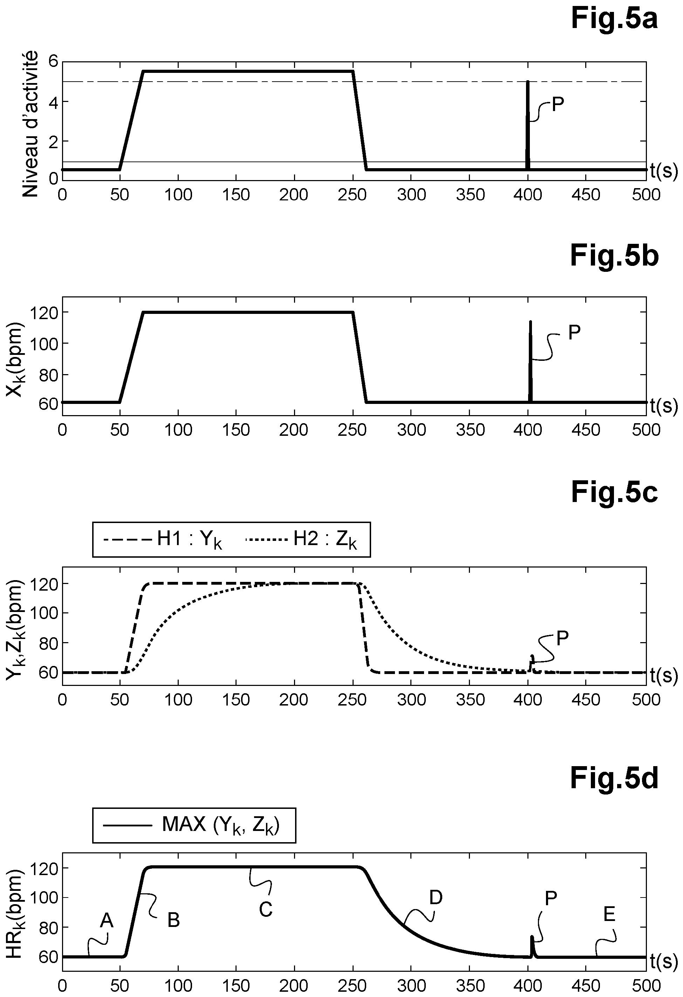

Les Figures 5a à 5d sont des chronogrammes illustrant des valeurs des signaux échantillonnés relevées à différents niveaux du module selon l'invention décrit ci-dessus, lors de variations détectées de l'activité du patient.

La Figure 5a montre un exemple de variation d'activité chez un patient, avec une brusque augmentation à t = 50 s, l'activité cessant à t = 250 s. On a également représenté en P sur cette figure un pic d'activité de valeur élevée mais très brève (correspondant à un saut, une toux, etc.), donc un pic parasite pour lequel il n'y a normalement pas lieu de modifier le rythme cardiaque.

La Figure 5b illustre la première valeur de rythme cardiaque cible $X_k$ en sortie de l'étage 110 de conversion activité vs. rythme cardiaque, qui suit donc de façon conforme les variations de l'activité, y compris le pic parasite P.

La Figure 5c montre les variations correspondantes des première et seconde valeur du signal cible, à savoir $Y_k$ en sortie du premier étage 120 de filtrage passe-bas H1 (en tiretés) et $Z_k$ en sortie du second étage 130 de filtrage passe-bas H2 (en pointillés). Comme on peut le voir, le filtrage H1 a permis de réduire considérablement l'intensité du pic parasite P tout en préservant la variation du niveau d'activité.

La Figure 5d montre la valeur de consigne finale $HR_k$ en sortie de l'étage combineur 140 : en A, en l'absence d'activité, c'est le signal $Z_k$ qui est sélectionné ; quand l'activité augmente brusquement en B c'est le signal $Y_k$ qui est sélectionné, et ce tant que l'activité demeure à un niveau élevé, en C. Lorsque l'activité décroît soudainement c'est en revanche la valeur $Z_k$ qui est sélectionnée, avec la constante de temps beaucoup plus longue, en D, permettant ainsi de répliquer le fonctionnement d'un cœur chez un patient sain, de façon comparable à ce qui avait été expliqué et illustré Figure 3a.

La Figure 6 illustre les variations de la valeur de consigne $HR_k$ de la Figure 5d, sous une forme paramétrique rythme cardiaque vs. niveau d'activité.

Comme on peut le voir, le pic d'activité P produit sur la courbe une variation importante, mais cette variation ne conduit pas à un changement significatif du rythme cardiaque : dans cet exemple, le rythme n'augmente que de 60 à 70 bpm. En revanche, lorsque l'activité s'établit, en C, de façon durable à un niveau élevé le rythme cardiaque augmente rapidement à sa valeur maximale $HR_{max}$, ici de 120 bpm.

La manière de procéder que l'on vient de décrire, caractéristique de l'invention, présente de nombreux avantages.

En particulier, l'impact du calcul de la valeur de consigne sur la consommation énergétique des circuits est particulièrement faible, en particulier si l'on utilise pour le calcul seulement des valeurs entières (donc sans calcul en virgule flottante). De plus, ce calcul ne nécessite qu'un nombre très limité de cellules de mémoire, sans qu'il soit besoin de conserver un nombre important de données d'historique du signal d'activité ou du signal de rythme cardiaque.

On notera enfin que, dans une version simplifiée, il est possible de supprimer le premier étage de filtrage passe-bas 120, avec dans ce cas $Y_k = X_k$.

Ceci permet de simplifier encore plus les circuits, mais avec une moindre immunité aux bruits parasites du signal d'activité recueillis par le capteur accélérométrique.

Par ailleurs, dans une variante de mise en œuvre de l'invention, le calcul des valeurs cibles n'est pas effectué en termes de rythme cardiaque (c'est-à-dire de fréquence), mais en termes d'intervalle cardiaque (c'est-à-dire de durée), l'intervalle cardiaque correspondant typiquement à l'intervalle R-R entre deux complexes QRS successifs d'un battement cardiaque.

La fonction F de l'étage de conversion 110 sera alors une fonction décroissante et non plus croissante (l'intervalle cardiaque doit diminuer quand l'activité augmente), et l'étage combineur 140 sélectionnera le minimum (et non plus le maximum) des valeurs d'intervalle cardiaque filtrées en sortie des étages passe-bas 120 et 130.

## Revendications

**1.** Un module de calcul d'une valeur de rythme cardiaque, HR, de consigne ($HR_k$) destinée à asservir à l'activité d'un patient la fréquence des impulsions de stimulation (38) délivrées par un dispositif médical implantable actif (10),

dans lequel la valeur HR de consigne ($HR_k$) est déterminée en continu par traitement numérique d'un signal d'activité échantillonné ($ACT_k$) représentatif de l'activité instantanée du patient,
**caractérisé en ce qu'**il comprend :

- un étage de conversion (110), recevant en entrée une valeur courante du signal d'activité échantillonné ($ACT_k$) et délivrant en sortie une première valeur de HR cible ($X_k$) obtenue par application d'une fonction activité/rythme cardiaque (F(ACT)) prédéterminée à une valeur courante du signal d'activité échantillonné

$(ACT_k)$ ;
- un premier étage de filtrage numérique passe-bas (130), comprenant un filtre récursif apte à calculer, sur une première durée prédéterminée, une première moyenne mobile de la première valeur de HR cible $(X_k)$ délivrée par l'étage de conversion (110), donnant en sortie une seconde valeur de HR cible $(Z_k)$ ; et
- un étage combineur (140), recevant en entrée (i) la première valeur de HR cible $(X_k)$ délivrée par l'étage de conversion (110), et (ii) la seconde valeur de HR cible $(Z_k)$ délivrée par le premier étage de filtrage numérique passe-bas (130), et apte à déterminer le maximum des deux valeurs de HR cible reçues en entrée, donnant en sortie la valeur HR de consigne $(HR_k)$, pour asservir la fréquence de stimulation à l'activité du patient.

2. Un module de calcul d'une valeur d'intervalle cardiaque, RR, de consigne destinée à asservir à l'activité d'un patient la fréquence des impulsions de stimulation (38) délivrées par un dispositif médical implantable actif (10),

dans lequel la valeur RR de consigne est déterminée en continu par traitement numérique d'un signal d'activité échantillonné $(ACT_k)$ représentatif de l'activité instantanée du patient,
**caractérisé en ce qu'**il comprend :

- un étage de conversion (110), recevant en entrée une valeur courante du signal d'activité échantillonné $(ACT_k)$ et délivrant en sortie une première valeur de RR cible obtenue par application d'une fonction activité/intervalle cardiaque (F(ACT)) prédéterminée à une valeur courante du signal d'activité échantillonné $(ACT_k)$ ;
- un premier étage de filtrage numérique passe-bas (130), comprenant un filtre récursif apte à calculer, sur une première durée prédéterminée, une première moyenne mobile de la première valeur de RR cible délivrée par l'étage de conversion (110), donnant en sortie une seconde valeur de RR cible ; et
- un étage combineur (140), recevant en entrée (i) la première valeur de RR cible délivrée par l'étage de conversion (110), et (ii) la seconde valeur de RR cible délivrée par le premier étage de filtrage numérique passe-bas (130), et apte à déterminer le minimum des deux valeurs de RR cible reçues en entrée, donnant en sortie la valeur RR de consigne, pour asservir la fréquence de stimulation à l'activité du patient.

3. Le module de la revendication 1 ou de la revendication 2, dans lequel le module est intégré à un circuit ASIC.

4. Le module de la revendication 1 ou de la revendication 2, dans lequel le module comprend un microcontrôleur opérant sans mise en sommeil entre deux cycles cardiaques consécutifs.

5. Le module de la revendication 1 ou de la revendication 2, dans lequel le module comprend en outre :

- un second étage de filtrage numérique passe-bas (120), comprenant un filtre récursif apte à calculer, sur une seconde durée prédéterminée inférieure à la première durée prédéterminée, une seconde moyenne mobile de la première valeur de HR cible $(X_k)$ , ou respectivement la première valeur de RR cible, délivrée par l'étage de conversion (110),

de manière à éliminer des composantes de bruit haute fréquence parasites non représentatives de l'activité instantanée du patient,
et dans lequel l'étage combineur (140) et le premier étage de filtrage numérique passe-bas (130) reçoivent en entrée la première valeur de HR cible $(X_k)$, ou respectivement la première valeur de RR cible, après que celle-ci a été filtrée par le second étage de filtrage numérique passe-bas (120).

6. Le module de la revendication 1 ou de la revendication 2, dans lequel la fonction activité/rythme cardiaque prédéterminée, ou respectivement la fonction activité/intervalle cardiaque prédéterminée, (F(ACT)) est une fonction linéaire.

7. Le module de la revendication 1 ou de la revendication 2, dans lequel, pour déterminer le temps de récupération du patient après une période d'activité intense, la première durée prédéterminée pour le calcul de la première moyenne mobile par le premier étage de filtrage numérique passe-bas (130) est comprise entre 120 et 600 secondes.

8. Le module de la revendication 5, dans lequel, pour déterminer la réactivité de l'adaptation du rythme cardiaque, ou respectivement de l'intervalle cardiaque, tout en éliminant les bruits d'activité de très courte durée, la seconde durée prédéterminée pour le calcul de la première moyenne mobile par le second étage de filtrage numérique passe-bas (120) est comprise entre 1 et 2 secondes.

**9.** Le module de la revendication 1, de la revendication 2, ou de la revendication 5, dans lequel le filtre récursif du premier étage de filtrage numérique passe-bas (130) et/ou du second étage de filtrage passe-bas (120) est un filtre récursif exponentiel du 1er ordre.

**10.** Le module de la revendication 1, de la revendication 2, ou de la revendication 5, dans lequel le filtre récursif du premier étage de filtrage passe-bas (130) et/ou du second étage de filtrage passe-bas (120) est un filtre apte à calculer une moyenne mobile sans division ni multiplication numérique.

**11.** Le module de la revendication 10, dans lequel le filtre récursif du premier étage de filtrage passe-bas (130) et/ou du second étage de filtrage passe-bas (120) est un filtre apte à opérer par décalage de bits de la représentation numérique de la valeur de HR cible, ou respectivement de la valeur de RR cible, en entrée de l'étage de filtrage.

**12.** Un dispositif médical actif de type capsule autonome implantable (10) logeant, dans un corps de dispositif (12), un ensemble électronique comprenant :

  - un circuit de stimulation (38), apte à délivrer des impulsions de stimulation ;
  - un circuit (46, 34) apte à délivrer un signal représentatif de l'activité instantanée d'un patient porteur du dispositif ;
  - un module d'échantillonnage et d'extraction d'un signal d'activité échantillonné (ACT$_k$) à partir du signal représentatif de l'activité instantanée du patient ; et
  - un module (110-140) de calcul d'une valeur HR ou RR de consigne selon l'une des revendications 1 à 11, pour asservir à l'activité du patient la fréquence des impulsions délivrées par le circuit de stimulation (38).

**Patentansprüche**

**1.** Ein Modul zur Berechnung eines Herzrhythmus, HR,

  - Sollwerts (Hr$_k$), dazu bestimmt, die Frequenz der Stimulations-Impulse (38), die von einer aktiven implantierbaren medizinischen Vorrichtung (10) bereitgestellt werden, der Aktivität eines Patienten nachzuführen,

  wobei der HR-Sollwert (HR$_k$) durch digitale Verarbeitung eines abgetasteten Aktivitätssignals (ACT$_k$), das für die momentane Aktivität des Patienten repräsentativ ist, kontinuierlich ermittelt wird, **dadurch gekennzeichnet, dass** es umfasst:

    - eine Wandlerstufe (110), die als eine Eingabe einen aktuellen Wert des abgetasteten Aktivitätssignals (ACT$_k$) empfängt und als eine Ausgabe einen ersten HR-Zielwert (X$_k$) bereitstellt, der durch Anwendung einer vorher festgelegten Aktivität/Herzrhythmus-Funktion (F(ACT)) auf einen aktuellen Wert des abgetasteten Aktivitätssignals (ACT$_k$) erhalten wird;
    - eine erste Tiefpass-Digitalfilter-Stufe (130), die ein rekursives Filter umfasst, das in der Lage ist, über eine vorher festgelegte erste Dauer, einen ersten gleitenden Mittelwert des ersten HR-Zielwerts (X$_k$), der von der Wandlerstufe (110) bereitgestellt wird, zu berechnen, und als eine Ausgabe einen zweiten HR-Zielwert (Z$_k$) bereitstellt; und
    - eine Zusammenführungsstufe (140), die als eine Eingabe (i) den ersten HR-Zielwert (X$_k$), der von der Zusammenführungsstufe (110) bereitgestellt wird, und (ii) den zweiten HR-Zielwert (Z$_k$), der von der ersten Tiefpass-Digitalfilter-Stufe (130) bereitgestellt wird, empfängt und in der Lage ist, das Maximum aus den zwei als eine Eingabe empfangenen HR-Zielwerten zu ermitteln, und als eine Ausgabe den HR-Sollwert (HR$_k$) bereitstellt, um die Stimulations-Frequenz der Aktivität des Patienten nachzuführen.

**2.** Ein Modul zur Berechnung eines Herzintervall, RR,

  - Sollwerts, dazu bestimmt, die Frequenz der Stimulations-Impulse (38), die von einer aktiven implantierbaren medizinischen Vorrichtung (10) bereitgestellt werden, der Aktivität eines Patienten nachzuführen,

  wobei der RR-Sollwert durch digitale Verarbeitung eines abgetasteten Aktivitätssignals (ACT$_k$), das für die momentane Aktivität des Patienten repräsentativ ist, kontinuierlich ermittelt wird, **dadurch gekennzeichnet, dass** es umfasst:

    - eine Wandlerstufe (110), die als eine Eingabe einen aktuellen Wert des abgetasteten Aktivitätssignals

($ACT_k$) empfängt und als eine Ausgabe einen ersten RR-Zielwert bereitstellt, der durch Anwendung einer vorher festgelegten Aktivität/Herzintervall-Funktion (F(ACT)) auf einen aktuellen Wert des abgetasteten Aktivitätssignals ($ACT_k$) erhalten wird;

- eine erste Tiefpass-Digitalfilter-Stufe (130), die ein rekursives Filter umfasst, das in der Lage ist, über eine vorher festgelegte erste Dauer, einen ersten gleitenden Mittelwert des ersten RR-Zielwerts, der von der Wandlerstufe (110) bereitgestellt wird, zu berechnen, und als eine Ausgabe einen zweiten RR-Zielwert bereitstellt; und

- eine Zusammenführungsstufe (140), die als eine Eingabe (i) den ersten RR-Zielwert, der von der Wandlerstufe (110) bereitgestellt wird, und (ii) den zweiten RR-Zielwert, der von der ersten Tiefpass-Digitalfilter-Stufe (130) bereitgestellt wird, empfängt und in der Lage ist, das Minimum aus den zwei als eine Eingabe empfangenen RR-Zielwerten zu ermitteln, und als eine Ausgabe den RR-Sollwert bereitstellt, um die Stimulations-Frequenz der Aktivität des Patienten nachzuführen.

3. Das Modul nach Anspruch 1 oder nach Anspruch 2, wobei das Modul in eine ASIC-Schaltung integriert ist.

4. Das Modul nach Anspruch 1 oder nach Anspruch 2, wobei das Modul ein Mikrocontroller ist, der ohne Ruhezeit zwischen zwei aufeinanderfolgenden Herzzyklen betrieben wird.

5. Das Modul nach Anspruch 1 oder nach Anspruch 2, wobei das Modul ferner umfasst:

- eine zweite Tiefpass-Digitalfilter-Stufe (120), die ein rekursives Filter umfasst, das in der Lage ist, über eine vorher festgelegte zweite Dauer, die kleiner als die vorher festgelegte erste Dauer ist, einen zweiten gleitenden Mittelwert des ersten HR-Zielwerts ($X_k$), bzw. des ersten RR-Zielwerts, der von der Wandlerstufe (110) bereitgestellt wird, zu berechnen, derart, dass Hochfrequenz-Störgeräuschkomponenten eliminiert werden, die für die momentane Aktivität des Patienten nicht repräsentativ sind, und wobei die Zusammenführungsstufe (140) und die erste Tiefpass-Digitalfilter-Stufe (130) als eine Eingabe den ersten HR-Zielwert ($X_k$), bzw. den ersten RR-Zielwert, empfangen, nachdem dieser durch die zweite Tiefpass-Digitalfilter-Stufe (120) gefiltert wurde.

6. Das Modul nach Anspruch 1 oder nach Anspruch 2, wobei die vorher festgelegte Aktivität/Herzrhythmus-Funktion, bzw. die Aktivität/Herzintervall-Funktion, (F(ACT)) eine lineare Funktion ist.

7. Das Modul nach Anspruch 1 oder nach Anspruch 2, wobei, um die Erholungszeit für den Patienten nach einem Zeitraum intensiver Aktivität zu ermitteln, die vorher festgelegte erste Dauer für die Berechnung des ersten gleitenden Mittelwerts durch die erste Tiefpass-Digitalfilter-Stufe (130) zwischen 120 und 600 Sekunden beträgt.

8. Das Modul nach Anspruch 5, wobei, um die Reaktivität der Anpassung des Herzrhythmus, bzw. des Herzintervalls, unter gleichzeitiger Eliminierung der Aktivitätsgeräusche mit sehr kurzer Dauer zu ermitteln, die vorher festgelegte zweite Dauer für die Berechnung des ersten gleitenden Mittelwerts durch die zweite Tiefpass-Digitalfilter-Stufe (120) zwischen 1 und 2 Sekunden beträgt.

9. Das Modul nach Anspruch 1, nach Anspruch 2 oder nach Anspruch 5, wobei das rekursive Filter der ersten Tiefpass-Digitalfilter-Stufe (130) und/oder der zweiten Tiefpass-Digitalfilter-Stufe (120) ein exponentielles rekursives Filter 1. Ordnung ist.

10. Das Modul nach Anspruch 1, nach Anspruch 2 oder nach Anspruch 5, wobei das rekursive Filter der ersten Tiefpass-Digitalfilter-Stufe (130) und/oder der zweiten Tiefpass-Digitalfilter-Stufe (120) ein Filter ist, das in der Lage ist, einen gleitenden Mittelwert ohne digitale Division oder Multiplikation zu berechnen.

11. Das Modul nach Anspruch 10, wobei das rekursive Filter der ersten Tiefpass-Digitalfilter-Stufe (130) und/oder der zweiten Tiefpass-Digitalfilter-Stufe (120) ein Filter ist, der durch Bit-Verschiebung der digitalen Darstellung des HR-Zielwerts, bzw. des RR-Zielwerts, als eine Eingabe der Filterstufe betreibbar ist.

12. Eine aktive medizinische Vorrichtung vom Typ implantierbare autonome Kapsel (10), die, in einem Vorrichtungskörper (12), eine elektronische Anordnung aufnimmt, die Folgendes umfasst:

- eine Stimulationsschaltung (38), die in der Lage ist, Stimulations-Impulse (38) bereitzustellen;
- eine Schaltung (46, 34), die in der Lage ist, ein Signal bereitzustellen, das für die momentane Aktivität eines Patienten, der die Vorrichtung trägt, repräsentativ ist;

- ein Modul zur Abtastung und zur Extraktion eines abgetasteten Aktivitätssignals ($ACT_k$) aus dem Signal, das für die momentane Aktivität eines Patienten repräsentativ ist; und
- ein Modul (110-140) zur Berechnung eines HR- oder RR-Sollwerts nach einem der Ansprüche 1 bis 11, um die Frequenz der Stimulations-Impulse, die von der Stimulationsschaltung (38) bereitgestellt werden, der Aktivität des Patienten nachzuführen.

**Claims**

1. A module for calculating a cardiac rhythm, HR, setpoint value ($HR_k$), for controlling a rate of pacing pulses (38) issued by an active implantable medical device (10) based on a patient's activity,

   wherein the HR setpoint value ($HR_k$) is continuously determined by digital processing of a sampled activity signal ($ACT_k$) representative of a patient's instantaneous activity,
   **characterized by** comprising:

   - a conversion stage (110), receiving as an input a current value of the sampled activity signal ($ACT_k$) and outputting a first target HR value ($X_k$), obtained by application of a predetermined activity vs. cardiac rhythm function (F(ACT)) to a current value of the sampled activity signal ($ACT_k$);
   - a first low-pass digital filtering stage (130), comprising a recursive filter adapted to calculate, over a first predetermined duration, a first moving average of the first target HR value ($X_k$) issued by the conversion stage (110), thereby outputting a second target HR value ($Z_k$); and
   - a combiner stage (140), receiving as an input (i) the first target HR value ($X_k$) issued by the conversion stage (110), and (ii) the second target HR value ($Z_k$) issued by the first low-pass digital filtering stage (130), and adapted to determine a maximum of the first and second target HR values received as an input, thereby outputting the HR setpoint value ($HR_k$) to control the rate of pacing pulses based on the patient's activity.

2. A module for calculating a cardiac interval, RR, setpoint value, for controlling a rate of pacing pulses (38) issued by an active implantable medical device (10) based on a patient's activity,

   wherein the RR setpoint value is continuously determined by digital processing of a sampled activity signal ($ACT_k$) representative of the patient's instantaneous activity,
   **characterized by** comprising:

   - a conversion stage (110), receiving as an input a current value of the sampled activity signal ($ACT_k$) and outputting a first target RR value, obtained by application of a predetermined activity vs. cardiac interval function (F(ACT)) to a current value of the sampled activity signal ($ACT_k$);
   - a first low-pass digital filtering stage (130), comprising a recursive filter adapted to calculate, over a first predetermined duration, a first moving average of the first target RR value issued by the conversion stage (110), thereby outputting a second target RR value; and
   - a combiner stage (140), receiving as an input (i) the first target RR value issued by the conversion stage (110), and (ii) the second target RR value issued by the first low-pass digital filtering stage (130), and adapted to determine a minimum of the first and second target RR values received as an input, thereby outputting the RR setpoint value, to control the rate of pacing pulses based on the patient's activity.

3. The module of claim 1 or claim 2, wherein the module is integrated to an ASIC circuit.

4. The module of claim 1 or claim 2, wherein the module comprises a microcontroller operating without being put to sleep between two consecutive cardiac cycles.

5. The module of claim 1 or claim 2, wherein the module further comprises:

   - a second low-pass digital filtering stage (120), comprising a recursive filter adapted to calculate, over a second predetermined duration shorter than the first predetermined duration, a second moving average of the first target HR value ($X_k$), or respectively the first target RR value, issued by the conversion stage (110), thereby eliminating parasitic high-frequency noise components that are not representative of the patient's instantaneous activity,
   and wherein the combiner stage (140) and the first low-pass digital filtering stage (130) receive as an input the first

target HR value ($X_k$), or respectively the first target RR value, after the latter has been filtered by the second low-pass digital filtering stage (120).

6. The module of claim 1 or claim 2, wherein the predetermined activity vs. cardiac rhythm function, or respectively the predetermined activity vs. cardiac interval function, (F(ACT)), is a linear function.

7. The module of claim 1 or claim 2, wherein, to determine the patient's recovery time after a period of intense activity, the first predetermined duration for the first low-pass digital filtering stage (130) to calculate the first moving average is between 120 and 600 seconds.

8. The module of claim 5, wherein, to determine the reactivity of the cardiac rhythm, or respectively of the cardiac interval, adaptation, while eliminating activity noises of very short duration, the second predetermined duration for the second low-pass digital filtering stage (120) to calculate the first moving average is between 1 and 2 seconds.

9. The module of claim 1, claim 2, or claim 5, wherein the recursive filter of at least one of the first low-pass digital filtering stage(130) and the second low-pass digital filtering stage (120) is an exponential recursive filter of the 1st order.

10. The module of claim 1, claim 2, or claim 5, wherein the recursive filter of the first low-pass digital filtering stage (130) and/or the second low-pass digital filtering stage (120) is a filter adapted to calculate a moving average without division nor multiplication.

11. The module of claim 10, wherein the recursive filter of the first low-pass digital filtering stage (130) and/or the second low-pass digital filtering stage (120) is a filter adapted to operate by shifting bits of the digital representation of the HR value, or respectively of the target RR value, at the input of the filtering stage.

12. An active medical device (10) of the implantable autonomous capsule type which houses, in a device body (12), an electronic unit including:

    - a pacing circuit (38), adapted to issue pacing pulses;
    - a circuit (46, 34) adapted to issue a signal representative of the instantaneous activity of a patient wearing the device;
    - a module for sampling or extracting a sampled activity signal ($ACT_k$) from the signal representative of the patient's instantaneous activity; and
    - a module (110-140) according to any of claims 1 to 11 for computing a HR or RR setpoint value, to control a rate of the pacing pulses issued by the pacing circuit (38) based on the patient's activity.

# Fig.1

# Fig.2

# Fig.3a

# Fig.3b

# Fig.4

# Fig.6

**Fig.5a**

**Fig.5b**

**Fig.5c**

**Fig.5d**

**EP 4 656 234 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2019001829 A1, Cairdac

- US 20200147396 A1, Shelton